# FASCICULE DE BREVET EUROPEEN

(11) **EP 2 580 185 B1**
(45) Date de publication et mention de la délivrance du brevet: **16.07.2014**
(21) Numéro de dépôt: 11722108.5
(22) Date de dépôt: 31.05.2011
(51) Int. Cl.: C07C 231/02

(54) **PROCEDE DE PREPARATION DE COMPOSES ESTARAMIDES**
VERFAHREN ZUR HERSTELLUNG VON ESTERAMIDVERBINDUNGEN
PROCESS FOR PREPARING ESTERAMIDE COMPOUNDS

(30) Priorité: 09.06.2010 FR 1054536
(43) Date de publication de la demande: 17.04.2013
(73) Titulaire: Rhodia Operations, 93306 Aubervilliers (FR)
(72) Inventeur: VIDAL, Thierry, 69003 Lyon (FR); RACHED, Rabih, 69390 Millery (FR); GUGLIERI, Massimo, 98000 Monaco (MC)
(74) Mandataire: Blot, Philippe Robert Emile
(86) Numéro de dépôt international: PCT/EP2011/058985
(87) Numéro de publication internationale: WO 2011/154292

(56) Documents cités:
- WO-A1-2009/092795

## Description

La présente invention concerne un procédé de préparation de composés esteramides.

Plus particulièrement, l'invention est relative à un procédé de préparation de composés esteramides par réaction entre un diester et une amine.

Les composés esteramides sont connus pour leurs applications comme solvants notamment dans des applications phytosanitaires, comme décrit par exemple dans le document WO 2009/092795.

Il existe plusieurs voies d'accès aux dits composés esteramides.

Le document US 4588833 décrit un procédé de préparation d'esteramides par réaction à haute température, catalysée par le cobalt, d'un amide insaturé avec un alcool et du monoxyde de carbone.

Le document US 3417114 décrit dans l'exemple 9, un procédé de préparation simultanée d'un composé esteramide « DMGME » de formule : MeOOC-(CH2)₃-CONMe₂ et d'un composé diamide « TMG » de formule : Me₂NOC-(CH₂)₃-CONMe₂, suivie de la séparation par distillation de ces deux composés. Pour faire la réaction, de la diméthylamine gazeuse est mise à buller pendant 2h dans un milieu comprenant du glutarate de diméthyle préalablement purifié et une solution de méthylate de sodium. Les deux composés (DMGME et TMG) sont ensuite isolés par distillation à partir du mélange complexe obtenu.

Le document US 3288794 décrit le même procédé que le document US 3417114 ainsi que la préparation simultanée d'ester méthylique de N,N-diméthyl-adipamide et de N,N,N',N'-tétraméthyl-adipamide avec un mode opératoire similaire, suivie également d'une séparation par distillation.

Dans le cas des diesters, les procédés de l'art antérieur ci-dessus décrits, ne sont pas sélectifs en esteramide. La proportion de diamide est d'ailleurs souvent majoritaire et l'esteramide est considéré comme un sous-produit qui n'a pas totalement réagi.

En outre, pour de tels procédés, une étape de purification du diester préalablement à la réaction est nécessaire, ce qui complique le procédé.

Ce type de procédé nécessite également des traitements lourds et coûteux du milieu réactionnel après réaction pour isoler le diamide et l'esteramide, comme par exemple des distillations.

De plus, avec ces procédés de l'art antérieur, les produits bruts formés peuvent présenter une forte coloration jaune-orangée qui est gênante pour les utilisations ultérieures. Les produits sont donc soumis à des étapes supplémentaires de purification. Tous ces traitements de purification complexifient les procédés de fabrication des esteramides.

Pour tenter de palier le problème de la faible sélectivité en esteramide, il est courant de travailler à des températures basses, c'est-à-dire inférieures à la température ambiante, en refroidissant le milieu réactionnel. Cependant, l'abaissement de la température de réaction diminue significativement la cinétique de la réaction et donc la productivité du procédé.

Il existe donc un besoin de trouver un procédé de fabrication de composés esteramides à partir de diesters qui soit sélectif en esteramide. En outre, le procédé doit être facile à mettre en oeuvre dans une installation industrielle. Un autre impératif auquel doit répondre le procédé est que la cinétique de la réaction doit être grande. Enfin, le procédé de fabrication d'esteramide devrait être productif.

A cet effet, la présente invention propose un procédé de préparation d'un composé esteramide de formule (I) suivante :

R¹OOC-A-CONR²R³ (I)

comprenant une étape de réaction entre :
un composé diester de formule (II) suivante :

   R¹OOC-A-COOR¹ (II)
et une amine de formule (III) suivante :

   HNR²R³ (III)

   en présence d'un composé basique,
formules dans lesquelles :
- A est une liaison covalente ou un groupe alkylène divalent linéaire ou ramifié comprenant un nombre d'atomes de carbone allant de 1 à 12,
- R¹ est un groupe hydrocarboné éventuellement substitué, comprenant de 1 à 36 atomes de carbone,
- R² et R³, identiques ou différents, sont des groupes choisis parmi l'atome d'hydrogène et les groupes hydrocarbonés, éventuellement substitués, comprenant de 1 à 36 atomes de carbone,
- R² et R³ pouvant former ensemble un cycle comprenant l'atome d'azote auquel ils sont liés, ledit cycle étant le cas échéant substitué et/ou comprenant un hétéroatome supplémentaire, et
- R² et R³ n'étant pas simultanément des atomes d'hydrogène,
caractérisé par le fait que
- l'on co-additionne l'amine de formule (III) sous forme gazeuse et le composé basique sur le composé diester de formule (II),
- et que la réaction est conduite à une température supérieure ou égale à 30 °C.

Par « l'on co-additionne », on comprendra au sens de la présente invention, que les composés sont ajoutés sur le composé diester (II) simultanément mais au moyen d'alimentations indépendantes. Par « simultanément », on comprendra que les composés auront au moins une période d'addition commune, l'addition pouvant débuter et s'achever à des moments différents pour chaque composé. De façon préférée, l'addition des deux composés débutera au même moment.

Selon un mode avantageux de réalisation de l'invention, la durée de co-addition (période d'addition commune) est comprise entre 30 minutes et 6 heures, de préférence entre 30 minutes et 3 heures et encore plus préférentiellement entre 30 minutes et 2 heures.

Intervient donc dans le procédé de l'invention un composé diester de formule (II), qui présente avantageusement les caractéristiques ci-dessous.

Selon un mode avantageux de l'invention, dans les formules (I) et (II), A est un groupe alkylène divalent ramifié comprenant un nombre d'atomes de carbone allant de 2 à 12, de préférence allant de 3 à 6 atomes de carbone.

De façon préférée, dans les formules (I) et (II), les groupes R¹, identiques ou différents, sont des groupes hydrocarbonés comprenant de 1 à 16 atomes de carbone et pouvant porter un ou plusieurs substituants. Par « substituant » on entend, à titre illustratif et sans caractère limitatif, un groupe alkyle ayant de préférence de 1 à 4 atomes de carbone, alcoxy ayant de préférence de 1 à 4 atomes de carbone, hydroxy ou halogéno.

Préférentiellement, les groupes R¹, identiques ou différents, sont choisis parmi les groupes alkyle, alcényle, cycloalkyle, aryle et arylalkyle, lesdits groupes pouvant porter un ou plusieurs substituants.

Plus particulièrement R¹ est préférentiellement choisi parmi les groupes méthyle, éthyle, n-propyle, isopropyle, n-butyle, isobutyle, tertiobutyle, sec-butyle, n-pentyle, isopentyle, sec-pentyle, cyclopentyle, n-hexyle, isohexyle, sec-hexyle, cyclohexyle, méthylcyclohexyle, 2-éthylbutyle, 3-méthylpentyle, n-heptyle, isoheptyle, sec-heptyle, 3-méthylhexyle, 4-méthylhexyle, 1-éthylpentyle, 2-éthylpentyle, 3-éthylpentyle, n-octyle, isooctyle, 3-méthylheptyle, n-nonyle, n-décyle, undécyle, n-dodécyle, tridécyle, tétradécyle et pentadécyle.

Dans un mode particulièrement avantageux, R¹ est choisi parmi les groupes méthyle et éthyle.

Tout préférentiellement, le composé diester de formule (II) est un mélange de composés diesters de formules (II.1), (II.2) et (II.3) suivantes :

R¹OOC-CH(CH₃)-CH₂-CH₂-COOR¹ (II.1)

R¹OOC-CH(CH₂-CH₃)-CH₂-COOR¹ (II.2)

R¹OOC-CH₂-CH₂-CH₂-CH₂-COOR¹ (II.3)

avec R¹ tel que défini ci-dessus.

Le mélange de composés diesters de formule (11.1), (11.2) et (11.3) peut présenter la composition suivante :
- de 75 à 95 % en poids de composé de formule (II.1), de préférence de 85 à 95 % en poids,
- de 3 à 23 % en poids de composé de formule (11.2), de préférence de 4 à 14 % en poids,
- de 0,1 à 10 % en poids de composé de formule (11.3), de préférence de 0,1 à 3 % en poids.

Il est particulièrement préféré que pour le mélange de composés diesters de formule (II.1), (11.2) et (11.3) ci-dessus les groupes R¹ soient des groupes méthyle. Il peut s'agir notamment d'un mélange de diesters commercialisé par Rhodia sous la dénomination Rhodiasolv® IRIS.

Selon un mode avantageux de l'invention, le composé diester (II) est introduit pur, c'est-à-dire qu'il n'est pas mis en solution dans un solvant organique. Toutefois, il est possible que le composé diester (II) soit mis en solution dans un solvant organique. Selon un mode préféré de réalisation de l'invention, le solvant organique est choisi parmi les solvants organiques volatils, notamment les alcools et les éthers, de préférence parmi le méthanol, l'éthanol, le tétrahydrofurane (THF) et leurs mélanges. Plus préférentiellement, le solvant organique est le méthanol.

Intervient également dans le procédé de l'invention une amine de formule (III), sous forme gazeuse, qui présente avantageusement les caractéristiques ci-dessous.

De façon préférée, dans les formules (I) et (III), les groupes R² et R³, identiques ou différents, sont des groupes hydrocarbonés comprenant de 1 à 16 atomes de carbone pouvant porter un ou plusieurs substituants. Par « substituant » on entend, à titre illustratif et sans caractère limitatif, un groupe alkyle ayant de préférence de 1 à 4 atomes de carbone, alcoxy ayant de préférence de 1 à 4 atomes de carbone, hydroxy ou halogéno.

Préférentiellement, les groupes R² et R³, identiques ou différents, sont choisis parmi les groupes alkyle, alcényle, cycloalkyle, aryle et arylalkyle, lesdits groupes pouvant porter un ou plusieurs substituants.

Selon un premier mode de réalisation de l'invention, R² et R³, identiques ou différents, sont choisis parmi les groupes méthyle, éthyle, hydroxyéthyle, n-propyle, isopropyle, n-butyle, isobutyle, tertiobutyle, n-pentyle, isoamyle, hexyle et cyclohexyle. De préférence, R² et R³ sont choisis parmi les groupes méthyle, éthyle et hydroxyéthyle.

Selon un second mode de réalisation de l'invention, R² et R³ forment ensemble un cycle à 5 ou 6 atomes comprenant l'atome d'azote auquel ils sont liés, l'un des atomes du cycle pouvant être un autre hétéroatome, tel que par exemple l'oxygène. De préférence, R² et R³ forment ensemble un cycle étant choisi parmi une morpholine, une pipéridine et une pipérazine.

Le procédé de l'invention met en jeu un composé basique.

De préférence, le composé basique est un alcoxyde de métal alcalin ou alcalino-terreux, de préférence choisi parmi le méthylate de sodium, l'éthylate de sodium ou de potassium, le ter-butylate de potassium. Le composé basique peut également être choisi parmi les carbonates, notamment le carbonate de potassium ou de sodium ; ou les titanates d'alkyle, comme par exemple le titanate de butyle. Le composé basique peut également être un mélange de plusieurs composés ci-dessus cités. De préférence, le composé basique est le méthylate de sodium.

Selon un mode préféré de réalisation de l'invention, le composé basique est en solution dans un solvant organique.

Dans ce cas, il s'agira du même solvant que celui décrit plus haut pour le composé diester (II), de préférence un solvant organique choisi parmi le méthanol, l'éthanol, le tétrahydrofurane (THF) et leurs mélanges. De préférence, le solvant organique est le méthanol.

Généralement, le composé basique est en solution dans un solvant organique à une concentration en poids dans le solvant organique qui est avantageusement comprise entre 5 et 80 %, de préférence entre 10 et 50 % et encore plus préférentiellement entre 20 et 30 %.

Conformément à l'invention, on fait réagir le composé diester de formule (II) et l'amine de formule (III) en présence du composé basique, dans les proportions définies ci-après.

Avantageusement, la quantité d'amine (III) sous forme gazeuse introduite correspond à un ratio molaire par rapport au composé diester (II) allant de 1 à 1,5, de préférence de 1 à 1,2 et encore plus préférentiellement de 1 à 1,1.

Le composé basique est de préférence introduit à une concentration molaire par rapport au composé diester (II) comprise entre 0,01 et 20%, de préférence entre 3 et 10%.

Selon une autre caractéristique de l'invention, la réaction est conduite à une température supérieure ou égale à 30°C. De préférence, la réaction est conduite à une température supérieure ou égale à 50°C. De façon avantageuse, la réaction est conduite à une température comprise entre 30°C et 130°C, de préférence entre 40°C et 90°C et encore plus préférentiellement entre 45°C et 65°C. Les calories nécessaires au contrôle de cette température sont apportées soit par la réaction elle-même, soit par un moyen externe, par exemple de chauffage ou de refroidissement.

De façon avantageuse, la réaction est conduite à une pression comprise entre 1 et 10 bars absolus, de préférence entre 1 et 5 bars absolus et encore plus préférentiellement entre 1 et 3 bars absolus.

De façon préférée, la réaction est conduite dans des conditions anhydres, c'est-à-dire que l'on tolère jusqu'à 0,2% d'eau, de préférence jusqu'à 0,05 % d'eau.

Selon un mode préféré, la réaction est conduite dans des conditions inertes, par exemple au moyen d'un balayage avec un gaz inerte, notamment de l'azote.

D'un point de vue pratique, on co-additionne l'amine de formule (III) sous forme gazeuse et le composé basique sur le composé diester de formule (II), la température étant maintenue dans la zone de température précédemment définie.

Conformément à l'invention, on obtient en fin de réaction le composé esteramide de formule (I). Le mélange réactionnel en fin de réaction ne nécessite pas de traitement lourd de purification du composé esteramide pour le séparer du composé diamide. Seuls les composés volatils comme l'amine n'ayant pas réagi et le solvant peuvent être éliminés, notamment évaporés par exemple par distillation sous pression réduite. Le milieu peut éventuellement être traité par des opérations classiques connues de l'homme du métier, notamment des étapes de neutralisation, filtration, et lavage pour éliminer les sels formés en cours de réaction. Le composé esteramide de formule (I) ainsi obtenu présente une grande pureté et peut être directement utilisé dans les applications auxquelles il est destiné, par exemple comme solvant, notamment dans des applications phytosanitaires.

Le procédé selon l'invention peut être un procédé continu ou discontinu.

Le procédé selon l'invention présente de nombreux avantages. Il est tout particulièrement avantageux lorsqu'il est mis en oeuvre sur un mélange de composés diesters répondant aux formules (II.1), (11.2) et (11.3), par exemple Rhodiasolv®IRIS et à une température supérieure à 30°C.

Tout d'abord, il est très sélectif en esteramide, c'est-à-dire que la sélectivité en esteramide est supérieure à 90%, voire supérieure à 95%. De façon surprenante, il se forme moins de 5% de diamide malgré l'excès d'amine et une température de réaction supérieure ou égale à 30°C. De plus, le procédé selon l'invention permet de contrôler la quantité d'amine introduite au moyen du bullage et de la durée de réaction. Ainsi, il n'est pas nécessaire d'introduire l'amine en excès par rapport au diester. La réaction est également très rapide comparée à des réactions à plus faible température et sans excès d'amine. En effet, le procédé selon l'invention présente des cinétiques de réaction élevées et donc des temps de réaction très courts. En outre, aucune coloration significative n'est observée et le mélange réactionnel en fin de réaction ne nécessite pas de traitement lourd de purification du produit majoritaire, ce qui simplifie grandement le procédé et augmente sa productivité.

Les exemples qui suivent, illustrent l'invention sans toutefois la limiter.

### Exemples

### Exemple 1 comparatif - Diméthylamine (gaz) sur mélange Rhodiasolv® IRIS / méthylate de sodium - T = 20°C

Dans un réacteur double enveloppé parfaitement agité de 1 L, muni d'une agitation mécanique et préalablement séché, sont placés une solution de méthylate de sodium dans le méthanol (solution à 50 % poids : poids, 18,8 g) et 304 g d'un mélange de diesters d'acide 2-méthyl-glutarique, d'acide 2-éthyl-succinique et d'acide adipique commercialisé sous la dénomination Rhodiasolv® IRIS par Rhodia.

On constate que le milieu réactionnel se colore fortement en jaune orangé.

La température du milieu réactionnel est stabilisée à la consigne de +20°C et de la diméthylamine gazeuse est mise à buller pendant 2 heures dans le mélange réactionnel comprenant le méthylate de sodium et le mélange de diesters (addition de 82 g de diméthylamine). La température du milieu réactionnel est maintenue constante pendant la durée du bullage.

La température du milieu réactionnel est maintenue à +20°C jusqu'à ce que 96 % du mélange de diesters initialement introduit ait été consommé (durée de finition : 20 heures).

Le léger excès de diméthylamine est distillé sous pression réduite (~200 mbar) à une température comprise entre 20 et 50 °C. Le milieu réactionnel est alors neutralisé par ajout de la quantité suffisante d'acide phosphorique à 85 %. Les sels formés sont filtrés. Le solide formé est lavé par du méthanol puis les composés volatils (méthanol et diester non réagi) sont distillés sous vide.

Le produit est alors analysé par chromatographie en phase gazeuse.

Le tableau 1 ci-après rassemble les conditions et résultats de cet exemple 1 comparatif.

### Exemple 2 comparatif - Diméthylamine (gaz) sur mélange Rhodiasolv® IRIS / méthylate de sodium - T = 50°C

Dans un réacteur double enveloppé parfaitement agité de 1 L, muni d'une agitation mécanique et préalablement séché, sont placés une solution de méthylate de sodium dans le méthanol (solution à 50 % poids : poids, 18,8 g) et 304 g d'un mélange de diesters d'acide 2-méthyl-glutarique, d'acide 2-éthyl-succinique et d'acide adipique commercialisé sous la dénomination Rhodiasolv® IRIS par Rhodia.

On constate que le milieu réactionnel se colore fortement en jaune orangé.

La température du milieu réactionnel est stabilisée à la consigne de + 50°C et de la diméthylamine gazeuse est mise à buller pendant 2 heures dans le mélange réactionnel comprenant le méthylate de sodium et le mélange de diesters (addition de 82 g de diméthylamine). La température du milieu réactionnel est maintenue constante pendant la durée du bullage.

La température du milieu réactionnel est maintenue à +50°C jusqu'à ce que 96 % du mélange de diesters initialement introduit ait été consommé (durée de finition : 2 heures).

Le léger excès de diméthylamine est distillé sous pression réduite (~200 mbar) à une température comprise entre 20 et 50°C. Le milieu réactionnel est alors neutralisé par ajout de la quantité suffisante d'acide phosphorique à 85 %. Les sels formés sont filtrés. Le solide formé est lavé par du méthanol puis les composés volatils (méthanol et diester non réagi) sont distillés sous vide.

Le produit est alors analysé par chromatographie en phase gazeuse.

Le tableau 1 ci-après rassemble les conditions et résultats de cet exemple 2 comparatif.

### Exemple 3 - Diméthylamine (gaz) et méthylate de sodium en co-addition sur Rhodiasolv® IRIS

Dans un réacteur double enveloppé parfaitement agité de 1 L, muni d'une agitation mécanique et préalablement séché, on place 304 g d'un mélange de diesters d'acide 2-méthyl-glutarique, d'acide 2-éthyl-succinique et d'acide adipique commercialisé sous la dénomination Rhodiasolv® IRIS par Rhodia.

On ne constate pas de coloration significative du milieu réactionnel.

La température du milieu réactionnel est stabilisée à la consigne de +50°C et l'on co-additionne de la diméthylamine gazeuse (bullage dans le diester - addition de 82 g de diméthylamine) et une solution de méthylate de sodium dans le méthanol (coulée - solution à 50 % poids : poids, 18,8 g).

La durée de la co-addition est de 2 heures. La température du milieu réactionnel est maintenue constante pendant la durée de la co-addition

La température du milieu réactionnel est maintenue à +50°C jusqu'à ce que 96 % du mélange de diesters initialement introduit ait été consommé (durée de finition : 2 heures).

Le léger excès de diméthylamine est distillé sous pression réduite (~200 mbar) à une température comprise entre 20 et 50°C. Le milieu réactionnel est alors neutralisé par ajout de la quantité suffisante d'acide phosphorique à 85%. Les sels formés sont filtrés. Le solide formé est lavé par du méthanol puis les composés volatils (méthanol et diester non réagi) sont distillés sous vide.

Le produit est alors analysé par chromatographie en phase gazeuse.

Le tableau 1 ci-dessous rassemble les conditions et résultats des différents exemples 1C, 2C et 3.

**Tableau 1**

| Essai | T (°C) | Durée (h)^{(a)} | Esteramide (%)^{(b)} | Diamide (%)^{(b)} | Di/EA (%)^{(c)} | Coloration du milieu réactionnel brut |
|---|---|---|---|---|---|---|
| 1C | 20 | 20 | 95 | 3 | 3,1 | Jaune-orangé |
| 2C | 50 | 2 | 96,3 | 3,7 | 3,8 | Jaune-orangé |
| 3 | 50 | 2 | 96,5 | 2,9 | 3,0 | pas de coloration |

| | | | | | | |
|---|---|---|---|---|---|---|
| (a) Durée nécessaire à l'achèvement de la réaction (b) Composition en espèce citée à la fin du traitement (c) Di/EA = rapport des concentrations finales diamide/esteramide | | | | | | |

Il ressort des exemples ci-dessus que, de façon surprenante, le procédé selon l'invention (exemple 3) est très sélectif en esteramide (96,5 %) et il se forme moins de 3 % de diamide. En outre, la réaction selon l'invention est très rapide (2h) et que le produit obtenu n'est pas coloré, ce qui permet son utilisation directe, sans étape supplémentaire de purification, contrairement aux procédés des exemples comparatifs.

## Revendications

1. Procédé de préparation d'un composé esteramide de formule (I) suivante :
R¹OOC-A-CONR²R³ (I)
comprenant une étape de réaction entre :
un composé diester de formule (II) suivante :
R¹OOC-A-COOR¹ (II)
et une amine de formule (III) suivante :
HNR²R³ (III)
en présence d'un composé basique,
formules dans lesquelles :
- A est une liaison covalente ou un groupe alkylène divalent linéaire ou ramifié comprenant un nombre d'atomes de carbone allant de 1 à 12,
- R¹ est un groupe hydrocarboné éventuellement substitué, comprenant de 1 à 36 atomes de carbone,
- R² et R³ identiques ou différents, sont des groupes choisis parmi l'atome d'hydrogène et les groupes hydrocarbonés, éventuellement substitués, comprenant de 1 à 36 atomes de carbone,
- R² et R³ pouvant former ensemble un cycle comprenant l'atome d'azote auquel ils sont liés, ledit cycle étant le cas échéant substitué et/ou comprenant un hétéroatome supplémentaire, et
- R² et R³ n'étant pas simultanément des atomes d'hydrogène,
**caractérisé par le fait que**
- l'on co-additionne l'amine de formule (III) sous forme gazeuse et le composé basique sur le composé diester de formule (II),
- et que la réaction est conduite à une température supérieure ou égale à 30°C.

2. Procédé selon la revendication 1, dans lequel A est un groupe alkylène divalent ramifié comprenant un nombre d'atomes de carbone allant de 2 à 12, de préférence allant de 3 à 6 atomes de carbone.

3. Procédé selon la revendication 1 ou 2, dans lequel les groupes R¹, identiques ou différents, sont des groupes hydrocarbonés comprenant de 1 à 16 atomes de carbone pouvant porter un ou plusieurs substituants.

4. Procédé selon l'une des revendications 1 à 3, dans lequel les groupes R¹, identiques ou différents, sont choisis parmi les groupes alkyle, alcényle, cycloalkyle, aryle et arylalkyle, lesdits groupes pouvant porter un ou plusieurs substituants.

5. Procédé selon l'une quelconque des revendications 1 à 4, dans lequel R¹ est choisi parmi les groupes méthyle, éthyle, n-propyle, isopropyle, n-butyle, isobutyle, tertiobutyle, sec-butyle, n-pentyle, isopentyle, sec-pentyle, cyclopentyle, n-hexyle, isohexyle, sec-hexyle, cyclohexyle, méthylcyclohexyle, 2-éthylbutyle, 3-méthylpentyle, n-heptyle, isoheptyle, sec-heptyle, 3-méthylhexyle, 4-méthylhexyle, 1-éthylpentyle, 2-éthylpentyle, 3-éthylpentyle, n-octyle, isooctyle, 3-méthylheptyle, n-nonyle, n-décyle, undécyle, n-dodécyle, tridécyle, tétradécyle et pentadécyle.

6. Procédé selon l'une quelconque des revendications 1 à 5, dans lequel le composé diester de formule (II) est un mélange de composés diesters de formules (II.1), (II.2) et (II.3) suivantes :
R¹OOC-CH(CH₃)-CH₂-CH₂-COOR¹ (II.1)
R¹OOC-CH(CH₂-CH₃)-CH₂-COOR¹ (II.2)
R¹OOC-CH₂-CH₂-CH₂-CH₂-COOR¹ (II.3)
avec R¹ tel que défini à l'une quelconque des revendications 1 à 5.

7. Procédé selon la revendication 6, dans lequel le mélange de composés diesters de formule (11.1), (11.2) et (11.3) présente la composition suivante :
- de 75 à 95 % en poids de composé de formule (II.1), de préférence de 85 à 95 % en poids,
- de 3 à 23 % en poids de composé de formule (11.2), de préférence de 4 à 14 % en poids,
- de 0,1 à 10 % en poids de composé de formule (11.3), de préférence de 0,1 à 3 % en poids.

8. Procédé selon la revendication 6 ou 7, dans lequel les groupes R¹ sont des groupes méthyle.

9. Procédé selon la revendication 1, dans lequel les groupes R² et R³, identiques ou différents, sont choisis parmi les groupes alkyle, alcényle, cycloalkyle, aryle et arylalkyle, lesdits groupes pouvant porter un ou plusieurs substituants.

10. Procédé selon la revendication 9, dans lequel R² et R³, identiques ou différents, sont choisis parmi les groupes méthyle, éthyle, hydroxyéthyle, n-propyle, isopropyle, n-butyle, isobutyle, tertiobutyle, n-pentyle, isoamyle, hexyle et cyclohexyle.

11. Procédé selon la revendication 1, dans lequel R² et R³ forment ensemble un cycle à 5 ou 6 atomes comprenant l'atome d'azote auquel ils sont liés, l'un des atomes du cycle pouvant être un autre hétéroatome, tel que par exemple l'oxygène.

12. Procédé selon la revendication 11, dans lequel R² et R³ forment ensemble un cycle choisi parmi une morpholine, une pipéridine et une pipérazine.

13. Procédé selon l'une des revendications 1 à 12, dans lequel le composé basique est un alcoxyde de métal alcalin ou alcalino-terreux, de préférence choisi parmi le méthylate de sodium, l'éthylate de sodium ou de potassium, le ter-butylate de potassium ; le carbonate de potassium ou de sodium, les titanates d'alkyle et leurs mélanges.

14. Procédé selon l'une des revendications 1 à 13, dans lequel le composé basique est en solution dans un solvant organique.

15. Procédé selon la revendication 14, dans lequel le solvant organique est choisi parmi les alcools et les éthers, de préférence parmi le méthanol, l'éthanol, le tétrahydrofurane (THF) et leurs mélanges.

16. Procédé selon l'une des revendications 1 à 15, dans lequel la quantité d'amine (III) sous forme gazeuse introduite correspond à un ratio molaire par rapport au composé diester (II) allant de 1 à 1,5, de préférence de 1 à 1,2 et encore plus préférentiellement de 1 à 1,1.

17. Procédé selon l'une des revendications 1 à 15, dans lequel le composé basique est introduit à une concentration molaire par rapport au composé diester comprise entre 0,01 et 20 %, de préférence entre 3 et 10 %.

18. Procédé selon l'une des revendications 1 à 16, dans lequel la réaction est conduite à une température comprise entre 30 °C et 130°C, de préférence entre 40°C et 90°C et encore plus préférentiellement entre 45°C et 65°C.

## Patentansprüche

1. Verfahren zur Herstellung einer Esteramidverbindung der folgenden Formel (I):
R¹OOC-A-CONR²R³ (I)
das einen Reaktionsschritt umfasst zwischen:
einer Diesterverbindung der folgenden Formel (II):
R¹OOC-A-COOR¹ (II)
und einem Amin der folgenden Formel (III):
HNR²R³ (III)
in Anwesenheit einer basischen Verbindung, wobei in den Formeln:
- A eine kovalente Bindung oder eine lineare oder verzweigte divalente Alkylengruppe ist, die eine Anzahl von Kohlenstoffatomen zwischen 1 und 12 umfasst;
- R¹ eine Kohlenwasserstoffgruppe, gegebenenfalls substituiert, ist, die 1 bis 36 Kohlenstoffatome umfasst,
- R² und R³, identisch oder unterschiedlich, Gruppen sind, die ausgewählt sind aus dem Wasserstoffatom und den Kohlenwasserstoffgruppen, gegebenenfalls substituiert, die 1 bis 36 Kohlenstoffatome umfassen,
- R² und R³ zusammen einen Zyklus bilden können, der ein Stickstoffatom umfasst, an das sie gebunden sind, wobei der Zyklus gegebenenfalls substituiert ist und/oder ein zusätzliches Heteroatom umfasst, und
- R² und R³ nicht gleichzeitig Wasserstoffatome sind,
**gekennzeichnet durch** die Tatsache, dass
- das Amin der Formel (III) in Gasform und die basische Verbindung zu der Diesterverbindung der Formel (II) hinzugefügt werden,
- und dass die Reaktion bei einer Temperatur größer als oder gleich 30°C durchgeführt wird.

2. Verfahren nach Anspruch 1, bei dem A eine divalente verzweigte Alkylengruppe ist, die eine Anzahl von Kohlenstoffatomen zwischen 2 und 12, vorzugsweise zwischen 3 und 6 Kohlenstoffatome umfasst.

3. Verfahren nach Anspruch 1 oder 2, bei dem die Gruppen R¹, identisch oder unterschiedlich, Kohlenwasserstoffgruppen sind, die zwischen 1 bis 16 Kohlenstoffatome umfassen, die einen oder mehrere Substituenten tragen können.

4. Verfahren nach einem der Ansprüche 1 bis 3, bei dem die Gruppen R¹, identisch oder unterschiedlich, ausgewählt sind aus den Gruppen Alkyl, Alkenyl, Cycloalkyl, Aryl und Arylalkyl, wobei die Gruppen einen oder mehrere Substituenten tragen können.

5. Verfahren nach einem beliebigen der Ansprüche 1 bis 4, bei dem R¹ ausgewählt ist aus den Gruppen Methyl, Ethyl, n-Propyl, Isopropyl, n-Butyl, Isobutyl, tert-Butyl, sec-Butyl, n-Pentyl, Isopentyl, sec-Pentyl, Cyclopentyl, n-Hexyl, Isohexyl, sec-Hexyl, Cyclohexyl, Methylcyclohexyl, 2-Ethylbutyl, 3-Methylpentyl, n-Heptyl, Isoheptyl, sec-Heptyl, 3-Methylhexyl, 4-Methylhexyl, 1-Ethylpentyl, 2-Ethylpentyl, 3-Ethylpentyl, n-Octyl, Isooctyl, 3-Methylheptyl, n-Nonyl, n-Decyl, Undecyl, n-Dodecyl, Tridecyl, Tetradecyl und Pentadecyl.

6. Verfahren nach einem beliebigen der Ansprüche 1 bis 5, bei dem die Diesterverbindung der Formel (II) eine Mischung von Diesterverbindungen der folgenden Formeln (11.1), (11.2) und (11.3) ist:
R¹OOC-CH(CH₃)-CH₂-CH₂-COOR¹ (II.1)
R¹OOC-CH(CH₂-CH₃)-CH₂-COOR¹ (II.2)
R¹OOC-CH₂-CH₂-CH₂-CH₂-COOR¹ (II.3)
wobei R¹ so definiert ist, wie in einem beliebigen der Ansprüche 1 bis 5.

7. Verfahren nach Anspruch 6, bei dem die Mischung der Diesterverbindungen der Formel (11.1), (11.2) und (11.3) die folgende Zusammensetzung aufweist:
- von 75 bis 95 Gew.-% der Verbindung der Formel (11.1), vorzugsweise von 85 bis 95 Gew.-%,
- von 3 bis 23 Gew.-% der Verbindung der Formel (11.2), vorzugsweise von 4 bis 14 Gew.-%,
- von 0,1 bis 10 Gew.-% der Verbindung der Formel (11.3), vorzugsweise von 0,1 bis 3 Gew.-%.

8. Verfahren nach Anspruch 6 oder 7, bei dem die Gruppen R¹ Methylgruppen sind.

9. Verfahren nach Anspruch 1, bei dem die Gruppen R² und R³, identisch oder unterschiedlich, ausgewählt sind aus den Gruppen Alkyl, Alkenyl, Cycloalkyl, Aryl und Arylalkyl, wobei die Gruppen einen oder mehrere Substituenten tragen können.

10. Verfahren nach Anspruch 9, bei dem R² und R³, identisch oder unterschiedlich, ausgewählt sind aus den Gruppen Methyl, Ethyl, Hydroxyethyl, n-Propyl, Isopropyl, n-Butyl, Isobutyl, tert-Butyl, n-Pentyl, Isoamyl, Hexyl und Cyclohexyl.

11. Verfahren nach Anspruch 1, bei dem R² und R³ zusammen einen Zyklus mit 5 oder 6 Atomen bilden, das Stickstoffatom umfassend, an das sie gebunden sind, wobei eines der Atome des Zyklus ein anderes Heteroatom, wie beispielsweise Sauerstoff sein kann.

12. Verfahren nach Anspruch 11, bei dem R² und R³ zusammen einen Zyklus bilden, der ausgewählt ist aus Morpholin, Piperidin und Piperazin.

13. Verfahren nach einem der Ansprüche 1 bis 12, bei dem die basische Verbindung ein Alkoxid eines Alkali- oder Erdalkalimetalls ist, vorzugsweise ausgewählt aus Natriummethylat, Natrium- oder Kaliumethylat, Kalium-tert-Butylat, Kalium- oder Natriumcarbonat, den Alkyltitanaten und ihren Mischungen.

14. Verfahren nach einem der Ansprüche 1 bis 13, bei dem die basische Verbindung in Lösung in einem organischen Lösungsmittel ist.

15. Verfahren nach Anspruch 14, bei dem das organische Lösungsmittel ausgewählt ist aus den Alkoholen oder Ethern, vorzugsweise aus Methanol, Ethanol, Tetrahydrofuran (THF) und ihren Mischungen.

16. Verfahren nach einem der Ansprüche 1 bis 15, bei dem die eingeführte Menge des Amins (III) in Gasform einem Molverhältnis in Bezug auf die Diesterverbindung (II) zwischen 1 bis 1,5 entspricht, vorzugsweise zwischen 1 bis 1,2 und noch bevorzugter zwischen 1 und 1,1 entspricht.

17. Verfahren nach einem der Ansprüche 1 bis 15, bei dem die basische Verbindung in einer Molkonzentration in Bezug auf die Diesterverbindung zwischen 0,01 und 20%, vorzugsweise zwischen 3 und 10% eingeführt wird.

18. Verfahren nach einem der Ansprüche 1 bis 16, bei dem die Reaktion bei einer Temperatur zwischen 30°C und 130°C, vorzugsweise zwischen 40°C und 90°C und noch bevorzugter zwischen 45°C und 65°C durchgeführt wird.

## Claims

1. A process for preparing an esteramide compound of following formula (I):
R¹OOC-A-CONR²R³ (I)
comprising a reaction step between:
a diester compound of following formula (II):
R¹OOC-A-COOR¹ (II)
and an amine of following formula (III):
HNR²R³ (III)
in the presence of a basic compound,
formulae in which:
- A is a covalent bond or a linear or branched divalent alkylene group comprising a number of carbon atoms ranging from 1 to 12,
- R¹ is an optionally substituted hydrocarbon group, comprising from 1 to 36 carbon atoms,
- R² and R³, the same or different, are groups chosen from the hydrogen atom and the hydrocarbon groups, optionally substituted, comprising from 1 to 36 carbon atoms,
- R² and R³ being able together to form a ring comprising the nitrogen atom with which they are linked, the said ring possibly being substituted and/or comprising an additional heteroatom, and
- R² and R³ not simultaneously being hydrogen atoms,
**characterized by** the fact that :
- the amine of formula (III) in gaseous form and the basic compound are co-added to the diester compound of formula (II),
- and the reaction is conducted at a temperature of 30°C or higher.

2. The process according to claim 1, wherein A is a branched divalent alkylene group comprising a number of carbon atoms ranging from 2 to 12, preferably ranging from 3 to 6 carbon atoms.

3. The process according to claim 1 or 2, wherein the R¹ groups, the same or different, are hydrocarbon groups comprising from 1 to 16 carbon atoms possibly carrying one or more substituents.

4. The process according to one of claims 1 to 3, wherein the R¹ groups, the same or different, are chosen from the group consisting of the alkyl, alkenyl, cycloalkyl, aryl and arylalkyl groups, the said groups possibly carrying one or more substituents.

5. The process according to any of claims 1 to 4, wherein R¹ is chosen from the group consisting of the methyl, ethyl, n-propyl, isopropyl, n-butyl, isobutyl, tertiobutyl, sec-butyl, n-pentyl, isopentyl, sec-pentyl, cyclopentyl, n-hexyl, isohexyl, sec-hexyl, cyclohexyl, methylcyclohexyl, 2-ethylbutyl, 3-methylpentyl, n-heptyl, isoheptyl, sec-heptyl, 3-methylhexyl, 4-methylhexyl, 1-ethylpentyl, 2-ethylpentyl, 3-ethylpentyl, n-octyl, isooctyl, 3-methylheptyl, n-nonyl, n-decyl, undecyl, n-dodecyl, tridecyl, tetradecyl and pentadecyl groups.

6. The process according to any of claims 1 to 5, wherein the diester compound of formula (II) is a mixture of diester compounds of following formulae (II.1), (II.2) and (II.3):
R¹OOC-CH(CH₃)-CH₂-CH₂-COOR¹ (II.1)
R¹OOC-CH(CH₂-CH₃)-CH₂-COOR¹ (II.2)
R¹OOC-CH₂-CH₂-CH₂-CH₂-COOR¹ (II.3)
where R¹ is such as defined in any of claims 1 to 5.

7. The process according to claim 6, wherein the mixture of diester compounds of formula (II.1), (11.2) and (11.3) has the following composition:
- from 75 to 95 % by weight of the formula (II.1) compound, preferably 85 to 95 % by weight,
- from 3 to 23 % by weight of the formula (11.2) compound, preferably 4 to 14 % by weight,
- from 0.1 to 10 % by weight of the formula (11.3) compound, preferably 0.1 to 3 % by weight.

8. The process according to claim 6 or 7, wherein the R¹ groups are methyl groups.

9. The process according to claim 1, wherein the groups R² and R³, the same or different, are chosen from the group consisting of the alkyl, alkenyl, cycloalkyl, aryl and arylalkyl groups, the said groups possibly carrying one or more substituents.

10. The process according to claim 9, wherein R² and R³, the same or different, are chosen from the group consisting of the methyl, ethyl, hydroxyethyl, n-propyl, isopropyl, n-butyl, isobutyl, tertiobutyl, n-pentyl, isoamyl, hexyl and cyclohexyl groups.

11. The process according to claim 1, wherein R² and R³ together form a ring with 5 to 6 atoms, comprising the nitrogen atom with which they are linked, one of the atoms of the ring possibly being another heteroatom, such as oxygen for example.

12. The process according to claim 11, wherein R² and R³ together form a ring chosen from morpholine, piperidine and piperazine.

13. The process according to any one of claims 1 to 12, wherein the basic compound is an alkoxide of an alkaline metal or alkaline-earth metal, preferably chosen from among sodium methylate, sodium or potassium ethylate, potassium ter-butylate; potassium or sodium carbonate, alkyl titanates and mixtures thereof.

14. The process according to any one of claims 1 to 13, wherein the basic compound is in solution in an organic solvent.

15. The process according to claim 14, wherein the organic solvent is chosen from alcohols and ethers, preferably from methanol, ethanol, tetrahydrofuran (THF) and mixtures thereof.

16. The process according to any one of claims 1 to 15, wherein the added quantity of amine (III) in gaseous form corresponds to a molar ratio relative to the diester compound (II) ranging from 1 to 1.5, preferably from 1 to 1.2 and further preferably from 1 to 1.1.

17. The process according to any one of claims 1 to 15, wherein the basic compound is added at a molar concentration relative to the diester compound comprised from 0.01 to 20 %, preferably from 3 to 10 %.

18. The process according to any one of claims 1 to 16, wherein the reaction is conducted at a temperature comprised from 30°C to 130 ° C, preferably from 40 ° C to 90 ° C and further preferably from 45°C to 65°C.
